# EUROPÄISCHE PATENTANMELDUNG

(11) **EP 2 133 068 A1**
(43) Veröffentlichungstag der Anmeldung: **16.12.2009**
(21) Anmeldenummer: 08010842.6
(22) Anmeldetag: 13.06.2008
(51) Int. Cl.: A61K 9/14, A61K 31/167, A61K 31/192, A61K 31/415, A61K 31/55, A61K 47/32, A61K 47/34, A61K 47/36, A61K 47/38

(54) **Verfahren zur Auswahl eines geeigneten Hilfsstoffs für die Herstellung von festen Dispersionen für pharmazeutische Formulierungen**

(71) Anmelder: Ratiopharm GmbH, 89079 Ulm (DE)
(72) Erfinder: Albers, Jessica, 45149 Essen (DE); Kleinebudde, Peter, Dr., 40627 Düsseldorf (DE); Höltje, Hans-Dieter, Dr., 10119 Berlin (DE); Knop, Klaus, Dr., 40764 Langenfeld (DE); Schmitz, Birte, 47652 Weeze (DE); Kraahs, Peter, Dr., 79189 Bad Krozingen (DE); Schulze Nahrup, Julia, Dr., 82061 Neuried (DE); Alles, Rainer, 4125 Riehen (CH)
(74) Vertreter: Teipel, Stephan

(57) **Zusammenfassung**

Die vorliegende Erfindung betrifft ein Verfahren zur Auswahl eines geeigneten Hilfsstoffs für die Herstellung einer festen Dispersion, die einen in amorpher Form in den Hilfsstoff eingebetteten pharmazeutischen Wirkstoff enthält, sowie pharmazeutische Formulierungen, die eine solche feste Dispersion umfassen. Die Formulierungen enthalten eine Eudragit E Matrix, in der Naproxen, Ibuprofen, Oxcarbazepin, Paracetamol, Lidocain oder Etofyllin eingebettet ist. Alternativ wird eine Copovidon-Matrix für Paracetamol, Neproxen, Celecoxid und Etofyllin vorgeschlagen. In einer Isomalt-Matrix befinden sich Paracetamol oder Etofyllin.

## Beschreibung

Die vorliegende Erfindung betrifft ein Verfahren zur Auswahl eines geeigneten Hilfsstoffs für die Herstellung einer festen Dispersion, die einen in amorpher Form in den Hilfsstoff eingebetteten pharmazeutischen Wirkstoff enthält, sowie pharmazeutische Formulierungen, die eine solche feste Dispersion umfassen.

Zahlreiche Versuche wurden unternommen, um die Bioverfügbarkeit von Wirkstoffen, insbesondere schwer löslichen Wirkstoffen zu erhöhen. Eine Möglichkeit besteht darin, entsprechende Wirkstoffe in amorpher Form zur Verfügung zu stellen. Verbindungen, die in amorpher statt kristalliner Form vorliegen, sind in der Regel leichter löslich und verfügen dementsprechend auch über eine höhere Bioverfügbarkeit.

Bei pharmazeutischen Formulierungen, die den Wirkstoff in amorpher Form enthalten, besteht allerdings das Problem, dass der Wirkstoff oftmals dazu neigt, während der Herstellung der Formulierung oder deren Lagerung auszukristallisieren. Verschiedene Versuche wurden unternommen, den Wirkstoff in amorpher Form zur Verfügung zu stellen und dann ein entsprechendes Rekristallisieren des Wirkstoffs zu unterdrücken. Eine der bekannten Möglichkeiten besteht darin, den Wirkstoff in amorpher Form in eine feste Dispersion eines Polymers als Löslichkeitsverbesserer einzubetten. Hierdurch können glasartige Partikel erhalten werden, in denen der Wirkstoff in amorpher Form vorliegt und die, wenn sie einen Rekristallisationsinhibitor enthalten, über einen längeren Zeitraum stabil sind, d.h. in denen der Wirkstoff zumindest während der üblichen Lagerdauer nicht rekristallisiert. Entsprechende feste Dispersionen sowie verschiedene Verfahren zu deren Herstellung sind beispielsweise aus der WO 03/000294 A1 sowie der EP-A-1 027 886 bekannt.

Ein Problem bei der Herstellung entsprechender amorpher, unterkühlter/glasartiger Schmelzen ist die Auswahl eines geeigneten Hilfsstoffs zur Einbettung des pharmazeutischen Wirkstoffs. Bei der Untersuchung entsprechender Hilfsstoff-Wirkstoff-Mischungen hat sich gezeigt, dass sich nicht jeder Hilfsstoff mit einem vorgegebenen pharmazeutischen Wirkstoff zu einer gewünschten festen Dispersion verarbeiten lässt, oder sich eine feste Dispersion bildet, die jedoch nicht lagerstabil ist. Darüber hinaus hat sich gezeigt, dass sich einige Hilfsstoff-Wirkstoff-Mischungen zwar zu festen Dispersionen verarbeiten lassen, dies jedoch nur mit einer relativ geringen Wirkstoffbeladung. Dies führt zu unerwünscht großen Dosierungseinheiten, die nur schlecht oder nicht mehr geschluckt werden können, da für die Dispersion des Wirkstoffs eine große Menge Hilfsstoff benötigt wird. Außerdem besteht das Risiko, dass wenn der Wirkstoff nicht vollständig in der Hilfsstoffmatrix gelöst wird, die nicht gelösten Wirkstoffpartikel als Kristallisationskeime wirken und es somit während der Lagerung des Produktes zu einer Rekristallisation des Wirkstoffs kommen kann.

Ferner ergibt sich bei der Herstellung von festen Dispersionen das Problem, dass diese bei der Schmelzextrusion in Abhängigkeit vom gewählten Hilfsstoff dazu neigen, klebrig zu sein. Hierdurch kann es zu einem Verkleben der Extrusionsstränge und damit zu einem unbrauchbaren Produkt kommen.

Es besteht somit ein Bedürfnis danach, für einen gegebenen pharmazeutischen Wirkstoff einen geeigneten Hilfsstoff für die Herstellung einer festen Dispersion, in der der Wirkstoff in amorpher Form in dem Hilfsstoff eingebettet vorliegt, gezielt auswählen zu können. Darüber hinaus wäre es wünschenswert, vorhersagen zu können, mit wieviel Wirkstoff ein gegebener Hilfsstoff beladen werden kann, ohne die Lagerstabilität der festen Dispersion negativ zu beeinflussen. Schießlich wäre es wünschenswert, entsprechende feste Dispersionen mittels Schmelzextrusion herstellen zu können.

Es wurde überraschend gefunden, dass eine gezielte Auswahl aufgrund der Bestimmung von Schmelzenthalpien und Schmelzpunkten der Wirkstoff-Hilfsstoff-Mischungen und der reinen Wirkstoffe, der chemischen Strukturen der Wirk- und Hilfsstoffe sowie der Glasübergangstemperatur der festen Dispersion getroffen werden kann. Insbesondere die Schmelzenthalpien und Schmelzpunkte der Wirkstoffe sowie der Wirk- und Hilfsstoff-Mischungen lassen sich leicht bestimmen, sodass mit Hilfe der vorliegenden Erfindung aufgrund dieser Messdaten lagerstabile feste Dispersionen und deren Wirkstoffbeladbarkeit ohne die Notwendigkeit der Durchführung aufwendiger Lagerstabilitätsmessungen leicht vorhergesagt werden können.

Die vorliegende Erfindung betrifft somit die Verfahren gemäß den unabhängigen Ansprüchen sowie pharmazeutische Formulierungen, die mit diesen Verfahren hergestellt werden können. Insbesondere betrifft die vorliegende Erfindung ein Verfahren zur Auswahl eines geeigneten Hilfsstoffs für die Herstellung einer festen Dispersion, die einen in amorpher Form in den Hilfsstoff eingebetteten pharmazeutischen Wirkstoff enthält, wobei der Hilfsstoff so gewählt wird, dass in einem Diagramm, in dem der Quotient der Schmelzenthalpie der Wirkstoff-Hilfsstoff-Mischung zu der Schmelzenthalpie des Wirkstoffs gegen den Quotienten des Schmelzpunkts der Wirkstoff-Hilfsstoff-Mischung zu dem Schmelzpunkt des Wirkstoffs als Punkt X aufgetragen ist, die Distanz c zwischen diesem Punkt X und dem Punkt 1/1 ≥ 0,08 ist.

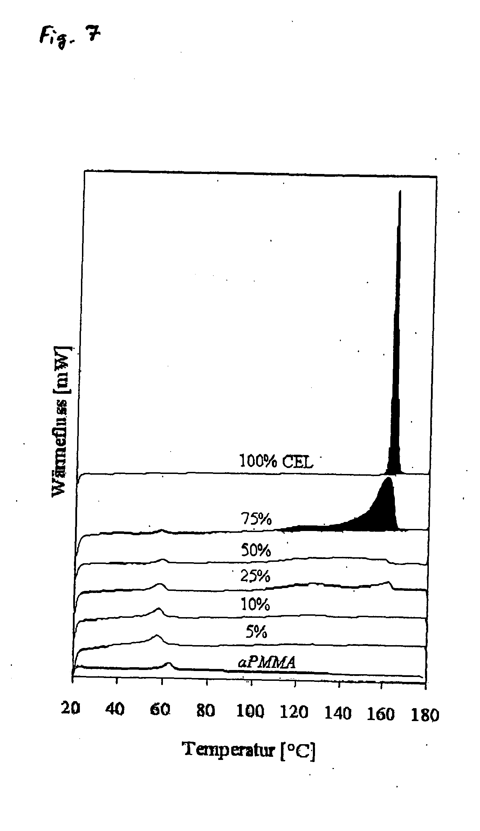

In dem erfindungsgemäßen Verfahren werden Schmelzenthalpie in J/g und Schmelzpunkt mittels DSC (Differential Scanning Calorometry) bei einer Aufheizrate von 10°C/min gemessen und dabei aus der ersten Aufheizkurve abgelesen. Als Schmelzpunkt wird die Onset-Schmelztemperatur gewählt. Als Schmelzenthalpie wird die Fläche des Wirkstoff-Schmelzpeaks definiert, welche aus Schmelz- und Lösungsenthalpie besteht (siehe schwarze Flächen in Figur 7). Zur Bestimmung der Schmelzenthalpie des Wirkstoffs in der Wirkstoff-Hilfsstoff-Mischung wird die Fläche des Wirkstoff-Schmelzpeaks auf 100 % Einwaage hochgerechnet.

Ob und wie gut sich ein Wirkstoff in einem Hilfsstoff löst, kann anhand von Schmelzenthalpie-Messungen mit Differential Scanning Calorimetrie (DSC) bestimmt werden. Dazu wird die Schmelzenthalpie des reinen Wirkstoffs und des Wirkstoffs in einer 1:1 Mischung mit einem Hilfsstoff bestimmt und der Quotient aus beiden Werten gebildet. Je kleiner der Quotient zwischen der Schmelzenthalpie des Wirkstoffs in der 1:1 Mischung mit dem Hilfsstoff und der Schmelzenthalpie des reinen Wirkstoffs ist, desto mehr Wirkstoff löst sich in dem Hilfsstoff. Die Bestimmung des Quotienten erlaubt eine grobe Abschätzung geeigneter Kombinationen von Wirkstoff und Hilfsstoff.

Schmelzenthalpien und deren Quotienten für eine Reihe von Wirkstoffen (WS) und deren 1:1 Mischung mit verschiedenen, beispielhaften Hilfsstoffen (HS) sind in der nachfolgenden Tabelle 1 wiedergegeben:

**Tabelle 1**

| **Wirkstoff** | **Hilfsstoff** | **Schmelzenthalpie reiner Wirkstoff [J/g]** | **Schmelzenthalpie Wirkstoff in 1:1 Mischung mit Hilfsstoff [J/g]** | **Quotient Enthalpie WS in HS / Enthalpie WS** |
|---|---|---|---|---|
| Ibuprofen | Eudragit E | 134 | 45 | 0,34 |
| Naproxen | Eudragit E | 143 | 50 | 0,35 |
| Oxcarbazepin | Eudragit E | 158 | 93 | 0,59 |
| Celecoxib | Eudragit E | 102 | 75 | 0,74 |
| Lidocain HCl | Eudragit E | 266 | 231 | 0,87 |
| Etofyllin | Eudragit E | 153 | 134 | 0,88 |
| Coffein | Eudragit E | 103 | 100 | 0,97 |
| Paracetamol | Eudragit E | 185 | 186 | 1,01 |
| Pentoxyfyllin | Eudragit E | 127 | 136 | 1,07 |
| Paracetamol | PVP VA64 | 185 | 89 | 0,48 |
| Naproxen | PVP VA64 | 143 | 73 | 0,51 |
| Celecoxib | PVP VA64 | 102 | 61 | 0,60 |
| Etofyllin | PVP VA64 | 153 | 127 | 0,83 |
| Etofyllin | Isomalt | 153 | 103 | 0,67 |
| Paracetamol | Isomalt | 185 | 170 | 0,92 |
| Naproxen | Isomalt | 143 | 138 | 0,97 |
| Celecoxib | Isomalt | 102 | 100 | 0,98 |

Es hat sich jedoch gezeigt, dass dieser Parameter alleine nicht ausreicht, um geeignete Wirkstoff-Hilfsstoff-Kombinationen zu wählen. Erst wenn ein zweiter Parameter hinzugezogen wird, nämlich der Quotient des Schmelzpunkts der Wirkstoff-Hilfsstoff-Mischung zu dem Schmelzpunkt des Wirkstoffs, lassen sich verwertbare Aussagen treffen.

In der nachfolgenden Tabelle 2 sind die Schmelzpunkte der Wirkstoffe und deren Mischungen mit Hilfsstoffen gemäß der vorstehenden Tabelle 1 sowie die sich daraus ergebenden Quotienten zusammengefasst:

**Tabelle 2**

| **Wirkstoff** | **Hilfsstoff** | **Schmelzpunkt Wirkstoff [°C] und [K]** | **Schmelzpunkt Wirkstoff in 1:1 Mischung mit Hilfsstoff [°C] und [K]** | **Quotient Schmelzpunkt WS in HS / Schmelzpunkt WS** |
|---|---|---|---|---|
| Celecoxib | Eudragit E | 162 / 435 | 97 / 370 | 0,85 |
| Naproxen | Eudragit E | 157 / 430 | 96 / 369 | 0,86 |
| Oxcarbazepin | Eudragit E | 231 / 504 | 201 / 474 | 0,94 |
| Paracetamol | Eudragit E | 169 / 442 | 155 / 428 | 0,97 |
| Ibuprofen | Eudragit E | 76 / 349 | 70 / 343 | 0,98 |
| Lidocain HCl | Eudragit E | 72 / 345 | 68 / 341 | 0,99 |
| Coffein | Eudragit E | 236 / 509 | 230 / 503 | 0,99 |
| Etofyllin | Eudragit E | 163 / 436 | 162 / 435 | 1,00 |
| Pentoxyfyllin | Eudragit E | 105 / 378 | 104 / 377 | 1,00 |
| Paracetamol | PVP VA64 | 169 / 442 | 122 / 395 | 0,89 |
| Naproxen | PVP VA64 | 157 / 430 | 113 / 386 | 0,90 |
| Etofyllin | PVP VA64 | 163 / 436 | 133 / 406 | 0,93 |
| Celecoxib | PVP VA64 | 162 / 435 | 136 / 409 | 0,94 |
| Etofyllin | Isomalt | 163 / 436 | 145 / 418 | 0,96 |
| Paracetamol | Isomalt | 169 / 442 | 164 / 437 | 0,99 |
| Celecoxib | Isomalt | 162 / 435 | 161 / 434 | 1,00 |
| Naproxen | Isomalt | 157 / 430 | 156 / 429 | 1,00 |

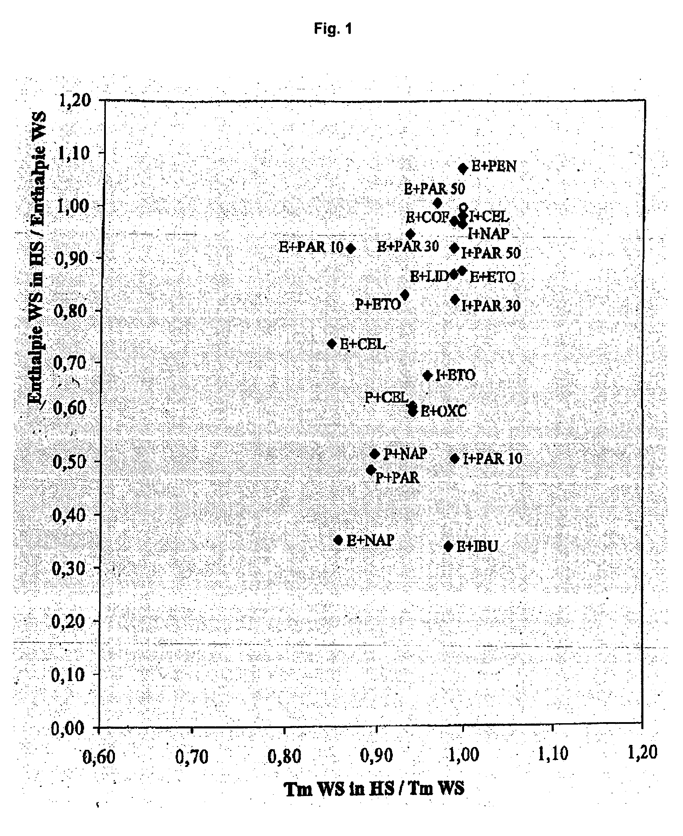

Trägt man den Quotienten der Schmelzenthalpien gegen den Quotienten der Schmelzpunkte auf, so ergibt sich ein Diagramm, aus dem man ablesen kann, ob und wie gut sich aus einem Wirkstoff und einem Hilfsstoff lagerstabile amorphe unterkühlte/glasartige Schmelzen mit 50% Wirkstoffbeladung herstellen lassen. Die Werte der vorstehenden Tabellen 1 und 2 sind in der anliegenden Figur 1 in einem entsprechenden Diagramm wiedergegeben. Nehmen die Quotienten "Tm Wirkstoff in Hilfsstoff/Tm Wirkstoff' bzw. "Enthalpie Wirkstoff in Hilfsstoff/Enthalpie Wirkstoff' den Wert 1 an (Kreis bei 1/1 im Diagramm in Figur 1), so liegt weder eine Schmelzpunkts- noch eine Enthalpieerniedrigung vor. Hier entstehen entweder kristalline oder zweiphasige amorphe Systeme. Darüber hinaus ist Paracetamol in den Hilfsstoffen Eudragit E und Isomalt nur zu einem geringeren Anteil gut löslich, so dass es bei dieser Auftragung unter die nichtmischbaren Systeme fällt. Je weiter das Stoffsystem vom Punkt 1/1 entfernt ist, desto besser können lagerstabile amorphe unterkühlte/glasartige Schmelzen hergestellt werden.

Erfindungsgemäß wurde nun gefunden, dass lagerstabile feste Dispersionen, die einen in amorpher Form in den Hilfsstoff eingebetteten pharmazeutischen Wirkstoff enthalten, mit Kombinationen erhalten werden, für die die Distanz c zwischen dem in dem Diagramm der Figur 1 eingetragenen Punkt X und dem Punkt 1/1 ≥ 0,08 ist. Vorzugsweise sollte die Distanz ≥ 0,15, am bevorzugtesten ≥ 0,30 sein. Die Distanz c lässt sich dabei nach dem Satz des Pythagoras gemäß a² + b² = c² berechnen, wobei a die Differenz zwischen 1 und dem Quotienten der Schmelzenthalpien und b die Differenz zwischen 1 und dem Quotienten der Schmelzpunkte ist.

Die Distanzen für die in Figur 1 gezeigten Kombinationen sind in der folgenden Tabelle 3 zusammengefasst, wobei folgende Abkürzungen verwendet werden CEL = Celecoxib, NAP = Naproxen, IBU = Ibuprofen, LID = Lidocain HCl, PAR = Paracetamol, OXC = Oxcarbazepin, PEN = Pentoxyfyllin, ETO = Etofyllin, COF = Coffein, E = Eudragit E, P = PVP VA64, I = Isomalt. Sofern nicht anders angegeben, weisen alle Proben eine Wirkstoffbeladung von 50 Gew.-% auf. Die Angabe "10" bzw. "30" gibt eine Wirkstoffbeladung von 10 Gew.-% bzw. 30 Gew.-% an.

**Tabelle 3**

| **Probe** | **Distanz** |
|---|---|
| E+NAP | 0,67 |
| E+IBU | 0,66 |
| P+PAR | 0,53 |
| P+NAP | 0,50 |
| I+PAR 10 | 0,50 |
| E+OXC | 0,42 |
| P+CEL | 0,41 |
| I+ETO | 0,33 |
| E+CEL | 0,30 |
| P+ETO | 0,18 |
| I+PAR 30 | 0,18 |
| E+PAR 10 | 0,15 |
| E+LID | 0,13 |
| E+ETO | 0,12 |
| I+PAR 50 | 0,08 |
| E+PAR 30 | 0,08 |
| E+PEN | 0,07 |
| I+NAP | 0,04 |
| E+PAR 50 | 0,03 |
| E+COF | 0,03 |
| I+CEL | 0,02 |

Wenn die Quotienten der Schmelzenthalpien und der Schmelzpunkte nicht nur für ein bestimmtes Mischungsverhältnis von Wirkstoff und Hilfsstoff sondern für unterschiedliche Mischungsverhältnisse bestimmt werden, kann mit Hilfe des erfindungsgemäßen Verfahrens darüber hinaus ermittelt werden, welche Wirkstoffbeladungen bei der Herstellung einer festen Dispersion möglich sind, ohne dass es zu einer Destabilisierung der erhaltenen festen Dispersionen kommt. Die festen Dispersionen sind nämlich solange stabil, wie die genannten Quotienten gegeneinander aufgetragen eine Distanz c zu dem Punkt 1/1 von ≥ 0,08 aufweisen.

Die vorliegende Erfindung betrifft somit auch ein Verfahren zur Bestimmung der möglichen Wirkstoffbeladung bei der Herstellung einer festen Dispersion, die einen in amorpher Form in einem Hilfsstoff eingebetteten pharmazeutischen Wirkstoff enthält, wobei in einem Diagramm die Quotienten der Schmelzenthalpien von Wirkstoff-Hilfsstoff-Mischungen bekannter Zusammensetzungen zu der Schmelzenthalpie des Wirkstoffs gegen die Quotienten der Schmelzpunkte dieser Wirkstoff-Hilfsstoff-Mischungen zu dem Schmelzpunkt des Wirkstoffs als Punkte aufgetragen werden und sich die mögliche Wirkstoffbeladung aus den Zusammensetzungen ergibt, deren Punkte eine Distanz c zu dem Punkt 1/1 von ≥ 0,08, vorzugsweise von ≥ 0,15, besonders bevorzugt von ≥ 0,30 aufweist.

Die erfindungsgemäßen Verfahren sind für praktisch jeden Wirkstoff geeignet, wobei solche Wirkstoffe bevorzugt sind, die in Wasser schwer löslich oder noch schlechter löslich sind. Unter in Wasser schwer löslichen Wirkstoffen werden vorliegend solche gemäß der Definition des Europäischen Arzneibuchs verstanden. Hierbei handelt es sich um Wirkstoffe, bei denen 1 Massenteil Substanz von 100-1000 Volumenteilen Lösungsmittel gelöst werden. Unter noch schlechter löslich werden solche Wirkstoffe verstanden, die mehr als 1000 Volumenteile Lösungsmittel für deren Auflösung benötigen. Entsprechende Wirkstoffe sind dem Fachmann bekannt und im Stand der Technik umfangreich beschrieben.

Die erfindungsgemäßen Verfahren eignen sich allerdings auch für besser und leicht lösliche Wirkstoffe, beispielsweise wenn diese mit schlecht bzw. langsam löslichen Hilfsstoffen zu Retardformulierungen verarbeitet werden sollen.

Die Verfahren der vorliegenden Erfindung eignen sich beispielsweise für Wirkstoffe aus den folgenden Wirkstoffgruppen: Acetylcholinesterase-Inhibitoren, Aknemittel, Analgetika, Antiphlogistika, Antiadiposita, Antiarrhythmika, Antiasthmatika, Antidementiva, Antibiotika (darunter Penicilline, Cephalosporine, Carbapeneme, Glycopeptide, Lincosamide, Aminoglycoside, Tetracycline, Makrolide, Nitroimidazol-Derivate, Gyrasehemmer, Sulfonamide), Antidepressiva, Antidiabetika, Antiemetika, Antiepileptika, Antihistaminika / Antiallergika, Antihypertonika, (darunter zentral angreifende α2-Agonisten, ACE-Hemmer, α-Sympatholytika, β-Sympatholytika, AT-Rezeptorantagonisten, Ca-Antagonisten), Antikoagulatien, Antikonvulsiva, Antimykotika, Antiparkinsonmittel, Antiprotozoika, Antipsoriatika, Antipsychotika, Antirheumatika, Aldosteron-Antagonisten, Alkoholentwöhnungsmittel, Mittel zur Behandlung der benignen Prostatahyperplasie, Biphosphonate, Cannabinoide, Cholesterol-Resorptionshemmer, COX-1-Inhibitoren, COX-2-Inhibitor, unspzifische COX-Inhibitoren, Diuretika, Endothelin-Rezeptorantagonist, Glucocorticoide, H2-Rezeptorantagonisten, Immunsupressiva, Inkontinenzmittel, Hormone, Mittel zur Behandlung der Hyperphosphatämie, Immunmodulatoren, Immunsuppressiva, Kontrazeptiva, Koronarmittel, Lipase-Inhibitoren, Lipid-Senker, Magen-Darm-Mittel, Malariamittel, Mucolytika, Multikinase-Inhibitoren, Mittel zur Behandlung der Multiplen Sklerose, Muskelrelaxantien, α-Sympatomimetika, Neuroleptika, Osteoporosemittel, Biphosphonate, Mittel zur Behandlung von HIV, Parasympatholytika, Phosphodiesterase-Hemmer, Protonenpumpeninhibitoren, Mittel zur Behandlung von Prostata-CA, Psychostimulantien, Renin-Hemmer, Sedativa, Spasmolytika, Steroidhormine, Thrombinhemmer, Tranquilantien, Triptane, Tyrosinkinase-Inhibitoren, Virustatika, Vitamine, insbesondere fettlösliche, Zytostatika (darunter Antimetaboliten, Alkylierende SubstanzenTopoisomerase-Hemmstoffe, Mitosehemmstoffe, Anthracycline, Antiestrogene, Antiandrogene, Aromatasehemmer).

Beispielsweise können folgende Wirkstoffe oder ihre pharmazeutisch verträglichen Salze eingesetzt werden: Abacavir, Acamprosat, Acarbose, Acebutolol, Aceclofenac, Acemetacin, Acetazolamid, Acetophenazin, Acetylcystein, Aciclovir, Acipimox, Acitretin Alendronat, Alfentanil, Alfuzosin, Alimemazin, Aliskiren, Almagat, Almotriptan, Alphacalcidol, Alpha Tocopherylacetat, Alprazolam, Alprenolol, Amantadin, Ambrisentan, Amifostin, Amilorid, Aminoglutethimid, Aminopentamid, Amiodaron, Amisulprid, Amitryptilin, Amlodipin, Amoxicillin, Amphetamin, Amphotericin B, Ampicillin, Amprenavir, Amrinon, Amsacrin, Anastrozol, Anidulafungin, Aprepitant, Aripiprazole, Atazanavir, Atenolol, Atomoxetin, Atorvastatin, Atovaquon, Atrasentan, Atrolactamid, Atropin, Avanafil, Azapropazon, Azathioprin, Azelastin, Azithromycin, Azosemid, Beclemid, Beclometason, Bemetizid, Benazepril, Bendamustin, Bendroflumethiazid, Benperidol, Benserazid, Benzquinamid, Bervastatin, Betahistin, Betamethason, Betaxolol, Bezafibrat, Bicalutamid, Bifeprunox, Bifonazol, Biperiden, Bisoprolol, Bleomycin, Bosentan, Brimonidin, Bromazepam, Bromocriptin, Bromoprid, Bromperidol, Brotizolam, Budesonid, Budipin, Bumetamid, Bupranolol, Buprenorphin, Bupropion, Buspiron, Busulfan, Butizid, Butyrophenon, Cabergolin, Calcipotriol, Calcitriol, Camptothecin, Candesartan, Capecitabin, Captopril, Carbamazepin, Carbidopa, Carboplatin, Carmustin, Carphenazin, Carvedilol, Cefaclor, Cefadroxil, Cefalexin, Cefamandol, Cefazolin, Cefepim, Cefetametpivoxil, Cefixim, Cefodizim, Cefotaxim, Cefotiam, Cefoxitin, Cefpodoximproxetil, Cefsulodin, Ceftazidim, Ceftibuten,Ceftriaxon, Cefuroxim, Cefuroximaxetil, Celecoxib, Celiprolol, Cerivastatin, Cetirizin, Chinin, Chlorambucil, Chlordiazepoxid, Chloroquin, Chlorphenoxamin, Chlorpromazin, Chlorprothixen, Chlorprotixen, Ciclesonide, Ciclopirox-Olamin, Ciclosporin, Cilastatin Cilazapril, Cilostazol, Cimetidin, Cinacalcet, Ciprofloxacin, Cisaprid, Cisplatin, Citalopram, Cladribin, Clarithromycin, Clavulansäure, Clemastin, Clinafloxacin, Clindamycin, Clobazam, Clobazam, Clodronat, Clofibrat, Clomethiazol, Clomipramin, Clonazepam, Clonidin, Clopamid, Clopenthixol, Clopidogrel, Clospirazin, Clothiapin, Clotrimazol, Clozapin, Codein, Colecalciferol, Cortison, Cromoglycinsäure, Cyclophosphamid, Cyproteronacetat, Cytarabin, Dabigatran, Dacarbazin, Dactinomycin, Dalvastatin, Dapoxetin, Darifenacin, Dasatinib, Daunorubicin, Delavirdin, Denaverin, Desipramin, Desloratadin, Desmopressin, Desogestrel, Desogestrel, Dexamethason, Dexchlorpheniramin, Dexetimid, Diamorphin, Diazepam, Dibenzepin, Diclofenac, Didanosin, Dienogest, Diethazin, Diflunisal, Dihydrocodein, Diltiazem, Dimenhydrinat, Dimenhydrinat, Dimethadion, Dimetinden, Diphenhydramin, Diphenylhydantoin, Dipyridamol, Disopyramid, Docetaxel, Dolasetron, Domperidon, Donepezil, Doxazosin, Doxepin, Doxorubicin, Doxycycllin, Doxylamin, Drofenin, Dronedaron, Droperidol, Drospirenon, Droxidopa, Duloxetin, Dutasterid, Econazol, Efavirenz, Eletriptan, Emtricitabin, Enalapril, Enfuvirtid, Enoxacin, Enoximon, Entacapone, Epirubicin, Eplerone, Eprosartan, Erlotinib, Erythromycin, Escitalopram, Esomeprazol, Estradiol, Estramustin, Ethinylestradiol, Ethopropazin, Ethosuximid, Ethylbenzhydramin, Etidronat, Etofibrat, Etofyllinclofibrat, Etoposid, Etoricoxib, Exemestan, Ezetimib, 5-FU (Fluorouracil), Famciclovir, Famotidin, Famotidin, Felbamat, Felbamat, Felodipin, Fendilin, Fenetyllin, Fenofibrat, Fenoterol, Fentanyl, Fenticonazol, Fesoterodin, Fexofenadin, Finasterid, Flecainid, Fleroxacin, Fluanison, Fluconazol, Flucytosin, Fludarabin, Flumazenil, Flunisolid, Flunitrazepam, Fluocortolon, Fluoxetin, Flupentixol, Fluphenaminsäure, Fluphenazin, Flupirtin, Flurazepam, Flurbiprofen, Fluspirilen, Flutamid, Fluticason, Fluvastatin, Fluvoxamin, Folinsäure, Formoterol, Fosinopril, Fosphenytoin, Frovatriptan, Furosemid, Gabapentin, Galantamin, Gallopamil, Gatifloxacin, Gefitinib, Gemcitabin, Gemfibrozil, Geprofloxacin, Gestoden, Glatiramer, Glibenclamid, Glibornurid, Gliclazid, Glimepirid, Glipizid, Gliquidon, Glisoxepid, Glucosamin, Goserelin, Granisetron, Griseofulvin, Guanfacin, Halofantrin; Haloperidol; Hydrochlorothiazid, Hydrocodon; Hydrocortison, Hydromorphon; Hydroxyzin; Ibandronat, Ibuprofen, Idarubicin, Ifosfamid, Imatinib, Imipenem, Imipramin, Imiquimod, Indapamid, Indinavir, Indiplon, Indometacin, Ipratropium, Ipratropiumbromid, Irbesartan, Irinotecan, Isoconazol, Isotretinoin; Isradipin, Itraconazol, Ixabepilon, Ketoconazol, Ketoprofen, Ketotifen, Labetalol, Lacidipin, Lamivudin, Lamotrigin, Lansoprazol, Lapatinib, Leflunomid, Lenalidomide; Lercanidipin, Letrozol; Leuprorelin, Levetiracetam, Levocetirizin, Levodopa, Levofloxacin, Levomepromazin, Levomethadon, Levonorgesterl, Licofelone, Lidocain, Liponsäure, Lisinopril, Lisurid, Lofepramin, Lomustin, Lonazolac, Lopinavir, Loracarbef; Loratadin, Lorazepam, Lormetazepam, Lornoxicam, Losartan, Lovastatin, Manidipin, Maprotilin, Maraviroc, Mebeverin, Meclozin, Mefenaminsäure, Mefloquin, Mefrusid, Meloxicam, Melperon, Memantine, Mephenytoin, Mephobarbital, Mepindolol, Mercaptopurin, Meropenem, Mesalazin, Mesuximid, Metamizol, Metformin, Methadon, Methamphetamin, Methotrexat; Methoxypromazin, Methsuximid, Methyldopa, Methylphenidat, Methylprednisolon, Metipranolol, Metoclopramid, Metoprolol, Metronidazol, Mexiletin, Mianserin, Miconazol, Midazolam, Miglitol, Milrinon, Minocyclin, Mirtazapin, Misoprostol, Mitiglinid, Mitomycin, Mitoxantron; Mizolastin, Moclobemid, Modafinil, Moexipril, Mofegilin, Molindon, Molsidomin, Mometason, Montelukast, Moperon, Morphin, Mosapramin, Moxifloxacin, Moxonidin; Mycophenolsäure, Nadolol, Nalbuphin, Naloxon, Naltrexon, Naproxen, Näratriptan, Narcobarbital, Natamycin, Nateglinid, Na-Valproate, Naxagolid, Nebivolol, Nedocromil, Nefazodon, Nefopam, Nelfinavir, Nevirapin, Nicardipin, Nicorandil, Nicotin, Nifedipin, Nilutamid; Nilvadipin, Nimesulid, Nimetazepam, Nimodipin, Nimorazol, Nimustin, Nisoldipin, Nitrazepam, Nitrendipin, Nitrezepam, NitroglycerinTTS; Nizatidin, Norfloxacin, Norgestimat, Nortryptilin, Nystatin, Octreotid, Ofloxacin, Olanzapin, Olmesartan, Olpadronat, Olsalazin, Omeprazol, Ondansetron, Opipramol, Orlistat ; Oseltamivir, Oxaceprol, Oxaflumazin, Oxaliplatin, Oxazepam, Oxcarbamazepin, Oxcarbazepin, Oxiconazol, Oxitropium; Oxitropiumbromid, Oxprenolol, Oxybutynin, Oxycodon; Paclitaxel, Paliperidone, Palonosetron, Pamidronat, Pantoprazol, Papaverin, Paracetamol, Paramethadion, Paroxetin, Pefloxacin, Pemetrexed, Pemolin, Penbutolol, Penciclovir, Penfluridol, Pentazocin, Pentostatin, Pentoxyfylin, Perazin, Pergolid, Perimethazin, Perindopril, Perphenazin, Pethidin, Phenacemid, Phenazon, Pheniramin, Phenobarbital, Phenotiazin, Phenprocoumon, Phensuximid, Phenylbutazon, Phenytoin, Pimecrolimus, Pimozid, Pindolol, Pioglitazon, Pipamperon, Piperacetazin, Piperacillin, Pirenzepin, Piretanid, Piribedil, Piritramid, Piroxicam, Pitavastatin, Pramipexol, Prasugrel, Pravastatin, Prazepam, Prednicarbat, Prednisolon, Prednison, Pregabalin, Primaquin, Primidon, Prochlorperazin, Progabid, Proguanil, Promazin, Promethazin, Propafenon, Propiverin, Propranolol, Propyphenazon, Prothipendyl, Pyrimethamin, Quetiapin, Quinapril, Rabeprazol; Racecadotril, Raloxifen, Ramelteon, Ramipril, Ranitidin, Ranolazin, Rasagilin, Reboxetin, Recuroniumbromid; Remacemid, Remifentanyl, Remoxiprid, Repaglinid, Retigabin, Ribavirin, Rifaximin, Rilmenidine, Riluzole, Rimonabant, Risedronat, Risperidon, Ritonavir, Rivaroxaban, Rivastigmin, Rizatriptan, Rofecoxib, Roflumilast, Ropinirol, Rosiglitazon, Rosuvastatin, Rotigotin, Roxatidinacetat, Roxithromycin, Rufinamid, Salmeterol, Saquinavir, Scopolamin, Selegilin, Sertindol, Sertraconazol, Sertralin, Sevelamer carbonate; Sevelamer, Sibutramin, Sildenafil, Simvastatin, Sirolimus, Sitagliptin, Solifenacin, Sorafenib; Sotalol, Sparfloxacin, Spiramycin, Spironolacton, Stavudin, Stiripentol, Strontiumranelat, Sufentanil, Sulbactam, Sulfacarbamid, Sulfadiazin, Sulfalen; Sulfamerazin, Sulfamethoxazol, Sulfasalazin; Sulforidazin, Sulpirid, Sultiam, Sumatriptan, Sunitinib, Tacrin, Tacrolimus, Tadalafil, Tamoxifen, Tamsulosin, Tazobactam; Teicoplanin, Telithromycin; Telmisartan, Temazepam, Temozolomid, Teniposid, Tenofovir, Tenoxicam, Terazosin, Terbinafin, Terfenadin, Tetrabenazin, Tetrazepam, Theophyllin, Thioguanin, Thioproperazin,Thioridazin, Thiotepa, Thiothixen, Thioxanthen, Tiagabin, Tianeptin, Tiaprid, Tiaprofensäure, Tibolon; Tilidin, Tiludronat, Timolol; Tinidazol; Tioconazol, Tiotropium, Tiropramid; Tizanidin, Tocainid, Tolbutamid, Tolterodin, Topiramat, Topotecan; Torasemid, Toremifen; Tramadol, Trandolapril, Tranylcypromin, Trazodon, Treosulfan, Tretinoin, Triamcinolon; Triamteren, Triazolam, Trichlormethiazid, Trifluoperazin, Trifluperidol, Triflupromazin, Triflusal, Trimetazidin, Trimethadion, Trimipramin, Tritoqualin, Trofosfamid, Tropicamid, Tropisetron; Trospiumchlorid, Urapidil, Valaciclovir, Valdecoxib, Valganciclovir, Valproat Natrium, Valproinsäure, Valsartan, Vancomycin; Vardenafil; Vareniclin, Vatalanib; Venlafaxin, Vigabatrin, Vildagliptin; Viloxazin; Vinblastin, Vincristin, Vinorelbin, Vitamine A,D,E,K, Voriconazol, Warfarin, Xipamid, Zafirliukast; Zalcitabin, Zaleplon, Zanamivir, Zidovudin, Ziprasidon, Zoledronat; Zolmitriptan, Zolpidem, Zonisamide, Zopiclon, Zotepin, Zuclopentihixol.

Beispielsweise eignen sich die in den vorstehenden Tabellen 1 und 2 genannten Wirkstoffe.

Die Wirkstoffe können einzeln oder in Kombination von zwei oder mehreren Wirkstoffen vorliegen. Ferner können die Wirkstoffe in ihrer freien Form, als pharmazeutisch verträgliche Salze oder als Cokristalle eingesetzt werden. Im Rahmen der vorliegenden Anmeldung wird unter dem Begriff "Wirkstoff' daher der Wirkstoff oder ein pharmazeutisch verträgliches Salz davon verstanden.

Erfindungsgemäß wird der Wirkstoff in einer festen Dispersion eingebettet, so dass er nach Einbettung in amorpher Form vorliegt. Unter "fester Dispersion" wird dabei eine Dispersion des amorphen Wirkstoffs in einer Hilfsstoffmatrix verstanden. Vorzugsweise ist der amorphe Wirkstoff molekular dispers in der Hilfsstoffmatrix verteilt. In diesem Fall handelt es sich bei der festen Dispersion um eine feste Lösung.

Als Hilfsstoffe können in dem Verfahren der vorliegenden Erfindung jeder bekannte Hilfsstoff, insbesondere solche Hilfsstoffe, die zur Herstellung fester Dispersionen bekannt sind, eingesetzt werden. Beispielhaft seien folgende Hilfsstoffe genannt:

Ammonium-methacrylat-copolymer, Poly(dimethylaminoethylmethacrylatco-methacrylat Ester), Poly(methyl acrylat-co-methyl methacrylat-co-methacrylsäure), Poly(methycrylsäureco-methyl methacrylat) 1:2, Hydroxypropylcellulose, Ethylcellulose, Celluloseacetatbutyrat, Celluloseacetatphthalat, Poly(ethylenoxid), Poly(ethylenglycol), Poly(vinylpyrrolidon), Poly(vinylacetat), Hydroxypropylmethylcellulose-phthalat, Polyvinylpyrrolidon-co-vinylacetat, Hydroxypropylmethylcellulose, Hydroxypropylmethylcellulose-acetatsuccinat, Poly(lactid-coglycolid), Polyvinylalkohol, Chitosanlactat, Pectin, Carbomer, Polycarbophil, Poly(ethyleneco-vinylacetat), Polyethylene, Poly(vinylacetat-co-methacrylsäure), Polycaprolacton, Carnaubawachs, Ethylenvinylacetat-copolymer, Glycerolpalmitostearat, Stärke, Maltodextrin, Isomalt, Zitronensäure, Natriumbicarbonat, Polyvinylalkohol-polyethylenglycol-copolymer.

Die Hilfsstoffe können alleine oder als Mischung von zwei oder mehr Hilfsstoffen verwendet werden. Vorzugsweise enthält die feste Dispersion jedoch nur einen Hilfsstoff.

Besonders bevorzugte Hilfsstoffe sind:
a) Aminoalkyl-methacrylat Copolymer der mittleren molekularen Masse 150.000 (Eudragit E), ein alternierendes Copolymer bestehend aus 50% Dimethyl-amino-ethyl-methacrylat, 25% Methyl-methacrylat und 25% Butyl-methacrylat,
b) Copovidon der mittleren molekularen Masse 45.000-70.000, ein statistisches Copolymer, bestehend aus 6 Teilen Vinylpyrrolidon und 4 Teilen Vinylacetat (PVP VA64), und
c) Isomalt.

In einer besonders bevorzugten Ausführungsform besteht die feste Dispersion aus dem Wirkstoff oder einem pharmazeutisch verträglichen Salz davon und einem Hilfsstoff. Zusätzliche Hilfsstoffe wie Weichmacher, Füllmittel, etc. würden demgegenüber die Rekristallisationsneigung des amorphen Wirkstoffs erhöhen. Die vorliegende Erfindung ermöglicht somit einen Verzicht auf entsprechende, sonst übliche Zusätze und stellt dementsprechend besonders lagerstabile Formulierungen zur Verfügung. Soweit entsprechende Zusätze benötigt oder gewünscht werden, können diese später mit der festen Dispersion gemischt werden, ohne hierdurch die amorphe Form des Wirkstoffs zu destabilisieren.

In einem weiteren Aspekt der vorliegenden Erfindung soll die feste Dispersion vorzugsweise durch Schmelzextrusion herstellbar sein. Voraussetzung für die Herstellung der Schmelze im Extruder ist neben der grundsätzlichen Mischbarkeit zusätzlich eine nicht klebrige Konsistenz der Schmelze beim Verlassen der Düsenplatte, da die Extrudate sonst nicht als getrennte Stränge erstarren, sondern zu einer einzigen Masse zusammenkleben.

Es wurde nun gefunden, dass das Extrudat dann klebrig ist und sich daher für ein Schmelzextrusionsverfahren nicht eignet, wenn die Glasübergangstemperatur der festen Dispersion unterhalb von etwa 18°C liegt. Beispiele für Glasübergangstemperaturen verschiedener Wirkstoff-Hilfsstoff-Mischungen sind in der folgenden Tabelle 4 zusammengefasst:

**Tabelle 4**

| **Extrudat** | **Tg [°C]** | **Klebrigkeit** |
|---|---|---|
| Eudragit E + Naproxen | 31 | - |
| Eudragit E + Ibuprofen | -4 | + |
| Eudragit E + Oxcarbazepin | 22 | - |
| Eudragit E + Paracetamol | 27 | - |
| PVP VA64 + Celecoxib | 63 | - |
| PVP VA64 + Paracetamol | 30 | - |
| PVP VA64 + Etofyllin | 24 | - |

Die Mischung aus Ibuprofen und Eudragit E, die eine Glasübergangstemperatur von -4°C aufweist, ist aufgrund ihrer Klebrigkeit für die Herstellung von festen Dispersionen durch Schmelzextrusion somit weniger geeignet.

Pharmazeutische Wirkstoffe und Hilfsstoffe können darüber hinaus anhand ihrer Struktur und der vorhandenen funktionellen Gruppen charakterisiert werden. Dabei wird ihre Acidität bzw. Basizität durch pKs und pKb-Werte beschrieben. Starke Säuren (Basen) besitzen kleine pKs (pKb)-Werte, schwache Säuren (Basen) haben hingegen große Gleichgewichtsexponenten. Sauren Charakter haben Verbindungen, die z.B. Phenole, Carbonsäuren, Aminosäuren, Sulfonsäuren, Imide, Sulfonamide oder NH- oder CH-acide Gruppen enthalten. Basische Gruppen können z.B. aliphatische und aromatische Amine, Imine, Nitrile, Amidine und Guanidine darstellen.

Das alternierende Copolymer Eudragit E besitzt beispielsweise als funktionelle Gruppen zwei Carbonylgruppen sowie eine Dimethylamino-Gruppe. Da die beiden Carbonylgruppen sehr nahe an der Kohlenstoff-Kette sitzen, können mit diesen kaum Wechselwirkungen eingegangen werden. Deshalb ist der Hauptreaktionspartner für Wirkstoffe die Aminogruppe. Durch das freie Elektronenpaar am Stickstoff besitzt die Gruppe starke Basizität und kann mit Säuren in einer Säure-Base-Reaktion in der Schmelze wechselwirken. Deshalb lösen sich insbesondere Wirkstoffe mit saurer funktioneller Gruppe wie Carbonsäuren (Ibuprofen, Naproxen) und Sulfonamiden gut in der Eudragit E Schmelze. Dabei löst sich in der Schmelze umso mehr Wirkstoff, je kleiner sein pKa-Wert ist, also je stärker seine sauren Eigenschaften sind. Das Proton der Carboxylgruppe kann leicht an Basen abgegeben werden, weil die negative Ladung des resultierenden Carboxylat-lons über drei Atome delokalisiert ist und dies zu einer Stabilisierung der konjugierten Base COO⁻ führt. Die unmittelbare Nachbarschaft von Carbonylgruppen oder anderen elektronenziehenden Strukturelementen zum freien Elektronenpaar eines Stickstoffatoms wirkt sich stark basizitätsmindernd aus, so dass Celecoxib den pKa-Wert einer schwachen Säure besitzt.

Auch Oxcarbazepin hat schwach sauren Charakter, da die Methylengruppe in der 2-Kohlenstoffbrücke zwischen einem aromatischen Ring und einer Carbonylgruppe CH-Acidität besitzt.

Paracetamol ist bezogen auf die Phenolfunktion eine schwache Säure, wobei das deprotonierte Phenolat eine relativ starke Base darstellt. Die Amidgruppe ist deutlich weniger basisch als ein Amin, so dass hier der schwach saure Charakter überwiegt. Eine Abnahme der Schmelzenthalpie zeigt sich in der DSC nicht, jedoch sollte eine Wechselwirkung zwischen dem Phenol des Wirkstoffs und der Dimethylaminogruppe des Hilfsstoffs möglich sein.

Über Schmelzextrusion konnten mit Eudragit E als Hilfsstoff lagerstabile unterkühlte/glasartige Schmelzen mit 50% der Wirkstoffe Ibuprofen, Naproxen und Oxcarbazepin hergestellt werden. Mit Paracetamol konnte eine Beladung von 30% realisiert werden.

Copovidon ist demgegenüber ein statistisches Copolymer, das aus Vinylpyrrolidon und Vinylacetat besteht. Da das Polymer sowohl aus hydrophilen Carbonylgruppen als auch aus lipophilen Kohlenstoffketten aufgebaut ist, handelt es sich um eine Verbindung, die sowohl in hydrophilen als auch in lipophilen Stoffen löslich ist. Die enthaltenen Carbonylgruppen stellen die funktionellen Einheiten dar, die als Elektronendonatoren Wasserstoffbrücken zu Polyphenolen ausbilden können und Interaktionen mit phenolischen OH-Gruppen ergeben. Somit können insbesondere die phenolische Gruppe des Paracetamols, die Säuregruppen des Naproxens und Celecoxibs und in geringerem Maße die alkoholische Gruppe des Etofyllins eine Wechselwirkung mit den Carbonylgruppen eingehen. Da bei diesem Hilfsstoff neben den hydrophilen Wechselwirkungen zusätzlich auch van der Waals Kräfte wirken können, lösen sich die verwendeten Arzneistoffe im geschmolzenen Hilfsstoff zu einem höheren Anteil. So konnten lagerstabile unterkühlte/glasartige Schmelzen mit den Arzneistoffen Paracetamol, Celecoxib und Etofyllin hergestellt werden.

Isomalt ist ein Zuckeralkohol, der aufgrund seiner vielen OH-Gruppen stark hydrophile Eigenschaften hat. Somit lösen sich in diesem Hilfsstoff Wirkstoffe mit hydrophilem Charakter, wie Paracetamol und Etofyllin.

Die Erfindung betrifft sowohl die Herstellung fester Dispersionen, insbesondere in Form von Extrudaten, als auch die weitere Verarbeitung der festen Dispersionen zu einer pharmazeutischen Formulierung. Diese pharmazeutische Formulierung kann als an sich bekanntes Arzneimittel, insbesondere feste Arzneiform vorliegen. Diese kann beispielsweise ausgewählt sein aus Tabletten, orodispersiblen Tabletten, Schmelztabletten, Pellets, Hartgelatinekapseln, Dragees, Granulaten, etc. Pellets und Granulate können beispielsweise in Sachets oder Stick-Packs abgefüllt vorliegen. Es sind aber auch flüssige Arzneiformen wie Suspensionen umfasst.

Zur Herstellung von Extrudaten wird der Wirkstoff mit dem Hilfsstoff gemischt. Diese Mischung wird in einen Extruder eingebracht und dort zu einer homogenen Schmelze verarbeitet. Das Schneckenprofil sollte Knetblöcke beinhalten, da die durch die Knetblöcke erzeugten Scherkräfte zum Aufschmelzen der Mischung und somit auch zum Auflösen des Wirkstoffs in dem Hilfsstoff beitragen. Das Temperaturprofil wird so gewählt, dass die höchsten Temperaturen in demjenigen Zylinder eingestellt werden, in dem die Knetblöcke sitzen. Durch Variation der beiden mittleren Zylinder wird bei dem Prozess bestimmt, wie stark die Mischung aufgeschmolzen wird. Da der WS im HS löslich ist, liegt diese Temperatur je nach System mindestens wenige Grade oberhalb der Glasübergangstemperatur bzw. des Schmelzpunkts des HS und höchstens 5°C unterhalb des Schmelzpunkts des WS. Über die Temperatur der beiden letzten Zylinder vor der Düsenplatte kann die Viskosität der Schmelze reguliert werden. Dabei gibt es für jedes System eine Mindesttemperatur, da die Düse sonst verstopft. Die Extrusion erfolgt vorzugsweise bei einem Auslassdruck von ca. 20 bar bis ca. 100 bar, insbesondere von ca. 20 bar bis ca. 50 bar.

Das erhaltene Extrudat liegt typischerweise in unterschiedlich langen Strängen vor, die nach dem Austritt aus dem Extruder nach dem Abkühlen zufällig gebrochen werden. Diese können mit einer Mühle, z.B. einer Zentrifugalmühle zerkleinert werden, um Fraktionen bestimmter Partikelgröße zu erhalten. Je nach Mahlvorgang kann man dabei Partikel unterschiedlicher Größe herstellen. Typischerweise liegt die Partikelgröße bei 0-500 µm. Dies entspricht gleichzeitig der Partikelgröße der meisten eingesetzten Hilfsstoffe zur Herstellung fester Arzneiformen. Somit wird ein besseres Mischen des gemahlenen Extrudats mit den Hilfsstoffen ermöglicht. Allgemein setzen kleine Extrudatpartikel den WS schneller frei als grobe Partikel.

Die Schmelze verlässt den Extruder in Form von Strängen unterschiedlicher Länge, die als solche keine Arzneiform darstellen. Um die Schmelze zu einer gleichmäßig dosierten Arzneiform zu verarbeiten, muss das Extrudat vorher mit einer Mühle (z.B. einer Zentrifugalmühle) gemahlen werden. Der Mahlprozess hat auf die amorphen unterkühlten / glasartigen Schmelzen keinen Einfluss, so dass sich keine Kristallkeime bilden und das amorphe System stabil bleibt.

Die erhaltenen Extrudate können auf an sich bekannte Art und Weise zu Arzneimitteln, insbesondere festen Arzneiformen, weiterverarbeitet werden.

Erfindungsgemäß bevorzugt sind Festdosierungsformen, wie Pellets, Kapseln und Tabletten. Diese können beschichtet oder unbeschichtet sein. Tabletten können beispielsweise durch Trockenkompaktierung oder Direktverpressen der zerkleinerten, insbesondere gemahlenen festen Dispersion hergestellt werden. Es können sowohl schnell freisetzende als auch Retard-Arzneiformen hergestellt werden.

Pellets können beispielsweise durch Schneiden von Strängen beim Verlassen eines Extruders und anschließendes Abkühlen hergestellt werden. Dazu eignet sich insbesondere ein hinter den Extruder geschalteter Mikropelletizer. Zur Herstellung von Granulaten eignet sich beispielsweise ein hinter einem Extruder und ein Kühlband geschalteter Granulator, der die erstarrten Stränge in Granulate schneidet.

Zur Herstellung von Kapseln kann die feste Dispersion gemahlen und gegebenenfalls mit weiteren Hilfsstoffen, wie Füllstoffe und Fließregulierungsmittel gemischt und in Kapseln, beispielsweise Gelatinekapseln, abgefüllt werden.

Die Formulierung der festen Dispersion zu Tabletten erfolgt mit üblichen pharmazeutischen Hilfs- und Zusatzstoffen. Geeignete Hilfsstoffe sind in der Regel Füllstoffe, Bindemittel, Sprengmittel, Fließregulierungsmittel, Schmiermittel, Geschmacksstoffe sowie gegebenenfalls weitere Zusatzstoffe.

Die erfindungsgemäßen pharmazeutischen Formulierungen können beispielsweise 0-90 Gew.-% Hilfsstoffe enthalten.

Der Gehalt an Sprengmitteln kann im Bereich von 1-40 Gew.-%, vorzugsweise 20-30 Gew.-% liegen, abhängig vom verwendeten Sprengmittel, der Füllstoffe und der übrigen Zusatzstoffe.

Der Gehalt an Schmiermittel ist in der Regel im Bereich von 0,1-4 Gew.-% , vorzugsweise etwa 0,5 Gew.-%.

Die vorstehend genannten Hilfsstoffmengen beziehen sich jeweils auf das Gesamtgewicht der pharmazeutischen Formulierung.

Als Füllstoffe können eine oder mehrere Verbindungen verwendet werden, wie z.B. mikrokristalline Cellulose, Stärke, Cellulosepulver, Lactose, insbesondere sprühgetrocknete Lactose, Glucose, Mannitol und Sorbitol.

Geeignete Sprengmittel sind Stärke, insbesondere Maisstärke, Alginsäure und deren Salze und Derivate, wie Calciumalginat und Natriumalginat, Natriumcarboxymethylcellulose, Polyacrylsäure, quervernetztes Polyvinylpyrrolidon, vernetzte Natriumcarboxymethylcellulose, quervernetzte Natriumcarboxymethylstärke, niedrigsubstituierte Natriumcarboxymethylcellulose, Natriumhydrogencarbonat und Magnesiumperoxid.

Geeignete Schmiermittel sind beispielsweise Magnesiumstearat, Calciumbehenat, Glycerinmonostearat, Stearinsäure, hydrierte Pflanzenfette, PEG 4000, Natriumdodecylsulfat, Magnesiumdodecylsulfat und Talcum.

Als Fließregulierungsmittel eignet sich beispielsweise hochdisperses Siliciumdioxid.

Liegt die pharmazeutische Formulierung beispielsweise als Tablette oder Pellet vor, kann sie mit einem oder mehreren Beschichtungsmitteln mit einer oder mehreren Schichten filmbeschichtet sein. Verwendbare Beschichtungsmittel sind beispielsweise Filmbildner, wie Cellulosederivate (z.B. MC, HEC, HPC, HPMC, NaCMC), Methacrylate, Copovidon, PVP, Celluloseacetatphthalat, Hyroxypropylmethylcellulosephthalat, Carboxymethylethylcellulose, Polyvinylacetatphthalat, Schellack, Methacrylsäureester und Ethylcellulose.

Falls erforderlich, können den Filmen weitere Hilfsstoffe wie z.B. Weichmacher zugesetzt werden.

Falls für die Beschichtung Wasser eingesetzt wird, kann dies zu einer Kristallisation des Wirkstoffs in der festen Dispersion führen oder diese beschleunigen. In diesem Fall kann es wünschenswert sein, die Beschichtung als "Dry Coating" aufzubringen oder eine Zwischenschicht vorzusehen, die die feste Dispersion vor einer Berührung mit dem Lösungsmittel während der Beschichtung schützt. Gleiches gilt, wenn die äußere Beschichtung eine Substanz wie einen Weichmacher enthält, die sich nachteilig auf die Stabilität der amorphen Form des Wirkstoffs auswirken kann.

Das Tablettengewicht ist nicht besonders eingeschränkt, übliche Tabletten sind 100 bis 800 mg, z.B. 300 bis 400 mg.

Von den anliegenden Figuren zeigt
**Figur 1** ein Diagramm, in dem die Quotienten der Schmelzenthalpien von Wirkstoff-Hilfsstoff-Mischungen zu der Schmelzenthalpie des Wirkstoffs gegen die Quotienten der Schmelzpunkte dieser Wirkstoff-Hilfsstoff-Mischungen zu dem Schmelzpunkt des Wirkstoffs aufgetragen sind.
**Figur 2** die Röntgenpulverspektren von kristallinem Celecoxib (CEL) und von einer festen Dispersion aus PVP VA64 und 50 Gew.-% Celecoxib nach Lagerung für 0, 1 und 3 Monate bei Raumtemperatur und etwa 0 % relativer Feuchte.
**Figur 3** die Röntgenpulverspektren von kristallinem Naproxen (NAP) und einer festen Dispersion aus Eudragit E und 50 Gew.-% Naproxen nach Lagerung für 0, 1 und 3 Monate bei Raumtemperatur und etwa 0 % relativer Feuchte.
**Figur 4** die Röntgenpulverspektren von kristallinem Paracetamol (PAR) und einer festen Dispersion aus Eudragit E und 30 Gew.-% Paracetamol nach Lagerung für 0, 1 und 3 Monate bei Raumtemperatur und etwa 0 % relativer Feuchte.
**Figur 5** die Röntgenpulverspektren von kristallinem Paracetamol (PAR) und einer festen Dispersion aus PVP VA 64 und 50 Gew.-% Paracetamol nach Lagerung für 0, 1 und 3 Monate bei Raumtemperatur und etwa 0 % relativer Feuchte.
**Figur 6** die Röntgenpulverspektren von kristallinem Oxcarbazepin (OXC) und einer festen Dispersion aus Eudragit E und 50 Gew.-% Oxcarbazepin nach Lagerung über 0, 1 und 3 Monate bei Raumtemperatur und etwa 0 % relativer Feuchte.
**Figur 7** eine Beispielmessung der Schmelzenthalpie.

Die vorliegende Erfindung wird nachfolgend anhand von Beispielen erläutert, die jedoch nicht einschränkend sein sollen.

### Beispiele:

### Beispiel 1:

### Vorhersage der Löslichkeit eines WS in einem HS

Schmelzpunkte (Tm) und Schmelzenthalpien der WS und der WS in den WS-HS-Mischungen wurden mittels Differential Scanning Calorimetry (DSC) bestimmt. Es wurden Quotienten gebildet aus
1. Tm WS in HS / Tm WS
2. Enthalpie WS in HS / Enthalpie WS
und gegeneinander aufgetragen (siehe Figur 1). Ist keine Lösung des WS im HS messbar, so ergibt sich im Diagramm ein Punkt mit den Koordinaten 1/1. Die Entfernung jeder Probe vom Punkt 1/1 wurde über den Satz des Pythagoras berechnet (a²+b²=c²).

Als Ergebnis dieses Versuchs konnte festgestellt werden, dass sich umso mehr WS im HS löst, je weiter die Probe im Diagramm vom Punkt 1/1 entfernt ist.

### Beispiel 2:

### Vorhersage der maximal möglichen Wirkstoffbeladung

1. Paracetamol wurde zu 10, 30 und 50% mit Eudragit E gemischt und wie in Beispiel 1 mit DSC vermessen. Sowohl für den Schmelzpunkt als auch für die Schmelzenthalpie ergab sich eine stärkere Abnahme je kleiner der WS-Anteil in der Mischung war.

**Schmelzverhalten von Paracetamol-Eudragit E-Mischungen (Enthalpie)**

| **Wirkstoffanteil [%]** | **Schmelzenthalpie reines Paracetamol [J/g]** | **Schmelzenthalpie Paracetamol in Mischung mit Eudragit E [J/g]** | **Quotient Enthalpie WS in HS / Enthalpie WS** |
|---|---|---|---|
| 10 | 185 | 170 | 0,92 |
| 30 | 185 | 175 | 0,95 |
| 50 | 185 | 186 | 1,01 |

**Schmelzverhalten von Paracetamol-Eudragit E-Mischungen (Schmelzpunkt)**

| **Wirkstoffanteil [%]** | **Schmelzpunkt reines Paracetamol [°C/K]** | **Schmelzpunkt Paracetamol in Mischung mit Eudragit E [°C/K]** | **Quotient Schmelzpunkt WS in HS / Schmelzpunkt WS** |
|---|---|---|---|
| 10 | 169 / 442 | 112 / 385 | 0,87 |
| 30 | 169 / 442 | 141 / 414 | 0,94 |
| 50 | 169 / 442 | 155 / 428 | 0,97 |

2. Paracetamol wurde zu 10, 30 und 50% mit Isomalt gemischt und wie in Beispiel 1 mit DSC vermessen. Sowohl für den Schmelzpunkt als auch für die Schmelzenthalpie ergab sich eine stärkere Abnahme je kleiner der WS-Anteil in der Mischung war.

**Schmelzverhalten von Paracetamol-Isomalt-Mischungen (Enthalpie)**

| **Wirkstoffanteil [%]** | **Schmelzenthalpie reines Paracetamol [J/g]** | **Schmelzenthalpie Paracetamol in Mischung mit Isomalt [J/g]** | **Quotient Enthalpie WS in HS / Enthalpie WS** |
|---|---|---|---|
| 10 | 185 | 93 | 0,50 |
| 30 | 185 | 152 | 0,82 |
| 50 | 185 | 170 | 0,92 |

**Schmelzverhalten von Paracetamol-Isomalt-Mischungen (Schmelzpunkt)**

| **Wirkstoffanteil [%]** | **Schmelzpunkt reines Paracetamol [°C/K]** | **Schmelzpunkt Paracetamol in Mischung mit Isomalt [°C/K]** | **Quotient Schmelzpunkt WS in HS / Schmelzpunkt WS** |
|---|---|---|---|
| 10 | 169 / 442 | 163 / 436 | 0,99 |
| 30 | 169 / 442 | 163 / 436 | 0,99 |
| 50 | 169 / 442 | 164 / 437 | 0,99 |

Es konnte festgestellt werden, dass man anhand dieser Versuche die maximale Beladung des HS mit WS messen kann. Ist keine Löslichkeit des WS im HS detektierbar, so kann auch im Extruder kein entsprechendes transparentes Extrudat hergestellt werden.

### Beispiel 3:

### Verfahren zur Herstellung einer Arzneiform enthaltend Eudragit E und einen Wirkstoff mit einer sauren funktionellen Gruppe

Für die Schmelzextrusion wurden 50 Gew.-% Eudragit E und 50 Gew.-% Naproxen in einem Turbula-Mischer gemischt und über die Dosiereinheit in den Extruder dosiert. Die Extrusion erfolgte bei einem Auslassdruck von 20 bis 50 bar. Die Zylinder wurden vor dem Einsatz mehrere Stunden vorgewärmt.

Unter den gewählten Bedingungen wurden transparente Extrudate in Form von Strängen mit einem Durchmesser von ca. 1 mm erhalten, welche bei Raumtemperatur abgekühlt und gemahlen wurden.

### Beispiel 4:

### Verfahren zur Herstellung einer Arzneiform enthaltend Copovidon und einen Wirkstoff mit phenolischen OH-Gruppen

Für die Schmelzextrusion wurden 50 Gew.-% Copovidon und 50 Gew.-% Celecoxib in einem Turbula-Mischer gemischt und über die Dosiereinheit in den Extruder dosiert. Die Extrusion erfolgte bei einem Auslassdruck von 20 bis 50 bar. Die Zylinder wurden vor dem Einsatz mehrere Stunden vorgewärmt.

Unter den gewählten Bedingungen wurden transparente Extrudate in Form von Strängen mit einem Durchmesser von ca. 1 mm erhalten, welche bei Raumtemperatur abgekühlt und gemahlen wurden.

### Beispiel 5:

Analog Beispiel 4 wurden feste Dispersionen aus 70 Gew.-% Eudragit E und 30 Gew.-% Paracetamol, 50 Gew.-% PVP VA64 und 50 Gew.-% Paracetamol sowie 50 Gew.-% Eudragit E und 40 Gew.-% Oxcarbazepin hergestellt.

### Beispiel 6:

### Charakterisierung der Festkörpereigenschaften, Testung auf Transparenz / amorphen Zustand

Mittels Röntgendiffraktometrie und DSC wurde überprüft, ob die in den Beispielen 3 bis 5 erhaltenen festen Dispersionen Kristalle enthalten oder ob sie im amorphen Zustand vorliegen. Im Röntgendiffraktogramm zeigte sich ein Halo, das den amorphen Zustand beschreibt. In der DSC ergab sich ein einziger Glasübergang, der darauf hinweist, dass sich der WS im HS gelöst hat und die Dispersionen insgesamt im amorphen Zustand vorliegen.

### Beispiel 7:

### Stabilität der Extrudate

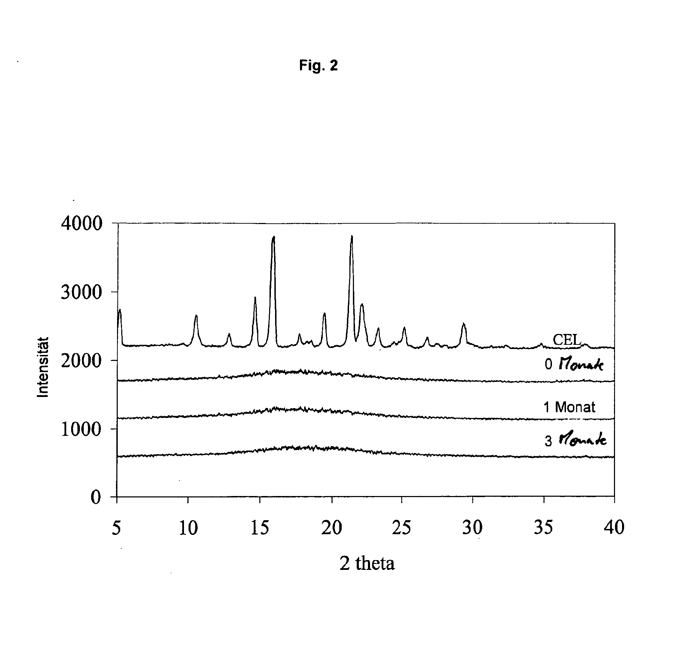

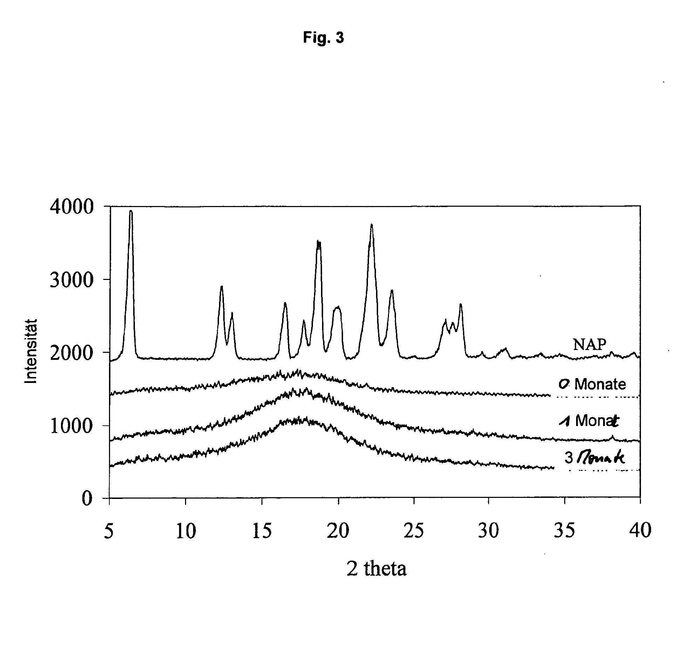

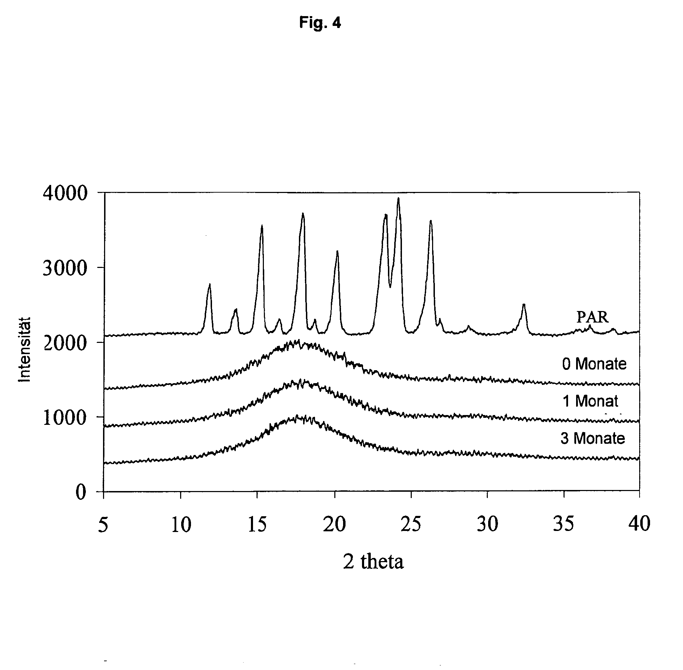

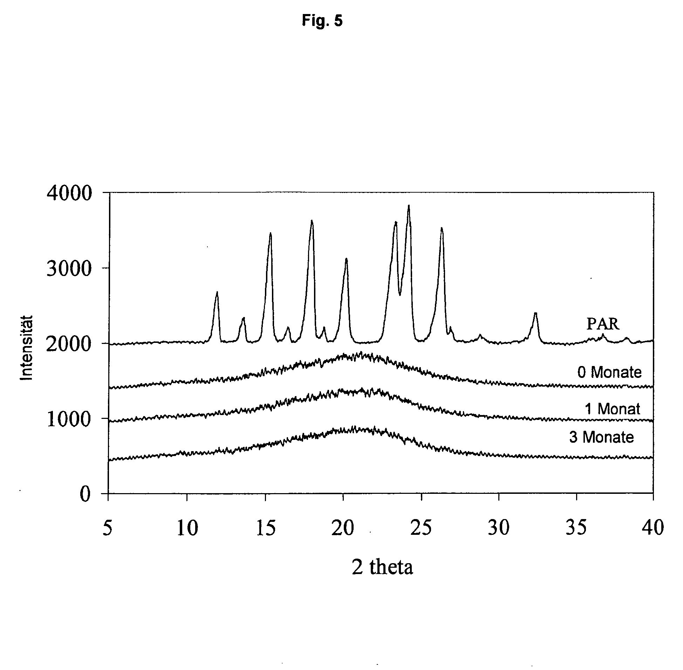

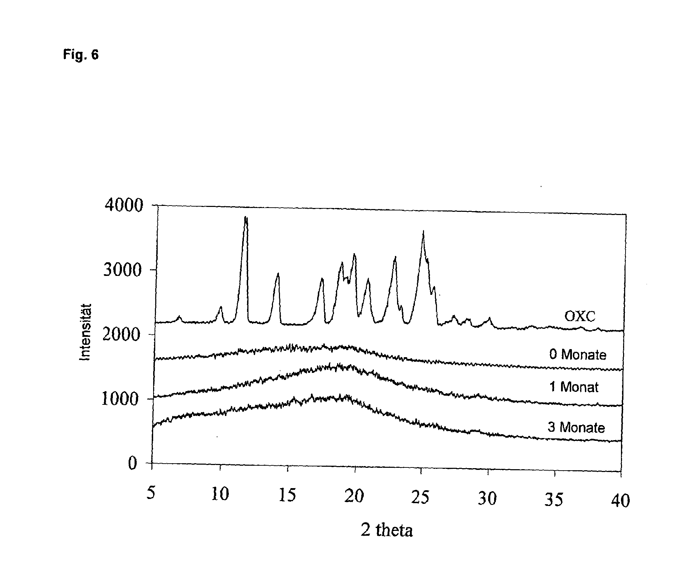

Die Extrudate der Beispiele 3 bis 5 wurden 3 Monate bei Raumtemperatur und etwa 0 % relativer Feuchte gelagert, um die Stabilität der amorphen Systeme zu untersuchen. Der Versuch zeigte, dass die transparenten Extrudate lagerstabil sind und nicht rekristallisieren (siehe Figuren 2-6).

## Patentansprüche

1. Verfahren zur Auswahl eines geeigneten Hilfsstoffs für die Herstellung einer festen Dispersion, die einen in amorpher Form in den Hilfsstoff eingebetteten pharmazeutischen Wirkstoff enthält, wobei der Hilfsstoff so gewählt wird, dass in einem Diagramm, in dem der Quotient der Schmelzenthalpie der Wirkstoff-Hilfsstoff-Mischung zu der Schmelzenthalpie des Wirkstoffs gegen den Quotienten des Schmelzpunkts der Wirkstoff-Hilfsstoff-Mischung zu dem Schmelzpunkt des Wirkstoffs als Punkt X aufgetragen ist, die Distanz c zwischen diesem Punkt X und dem Punkt 1/1 ≥ 0,08 ist.

2. Verfahren zur Bestimmung der möglichen Wirkstoffbeladung bei der Herstellung einer festen Dispersion, die einen in amorpher Form in einem Hilfsstoff eingebetteten pharmazeutischen Wirkstoff enthält, wobei in einem Diagramm die Quotienten der Schmelzenthalpien von Wirkstoff-Hilfsstoff-Mischungen bekannter Zusammensetzungen zu der Schmelzenthalpie des Wirkstoffs gegen die Quotienten der Schmelzpunkte dieser Wirkstoff-Hilfsstoff-Mischungen zu dem Schmelzpunkt des Wirkstoffs als Punkte aufgetragen werden und sich die mögliche Wirkstoffbeladung aus den Zusammensetzungen ergibt, deren Punkte eine Distanz c zu dem Punkt 1/1 von ≥ 0,08 aufweisen.

3. Verfahren nach Anspruch 1 oder 2, worin die Distanz c ≥ 0,30 ist.

4. Verfahren nach einem der vorhergehenden Ansprüche, wobei die feste Dispersion durch Schmelzextrusion herstellbar ist.

5. Verfahren nach Anspruch 4, wobei die feste Dispersion eine Glasübergangstemperatur von > 18°C aufweist.

6. Verfahren zur Auswahl eines geeigneten pharmazeutischen Wirkstoffs für die Herstellung einer festen Dispersion, die den in amorpher Form in Eudragit E eingebetteten Wirkstoff enthält, wobei der Wirkstoff so gewählt wird, dass er mindestens eine saure funktionelle Gruppe aufweist.

7. Verfahren zur Auswahl eines geeigneten pharmazeutischen Wirkstoffs für die Herstellung einer festen Dispersion, die den in amorpher Form in Copovidon eingebetteten Wirkstoff enthält, wobei der Wirkstoff so gewählt wird, dass er mindestens eine phenolische OH-Gruppe aufweist.

8. Verfahren zur Auswahl eines geeigneten pharmazeutischen Wirkstoffs für die Herstellung einer festen Dispersion, die den in amorpher Form in Isomalt eingebetteten Wirkstoff enthält, wobei der Wirkstoff so gewählt wird, dass er einen hydrophilen Charakter aufweist.

9. Pharmazeutische Formulierung, umfassend eine feste Dispersion, die nach einem der Verfahren der vorhergehenden Ansprüche erhältlich ist.

10. Pharmazeutische Formulierung, umfassend eine feste Dispersion, die einen in amorpher Form in einen Hilfsstoff eingebetteten pharmazeutischen Wirkstoff enthält, wobei der Hilfsstoff und der Wirkstoff so gewählt sind, dass in einem Diagramm, in dem der Quotient der Schmelzenthalpie der Wirstoff-Hilfsstoff-Mischung zu der Schmelzenthalpie des Wirkstoffs gegen den Quotienten des Schmelzpunkts der Wirkstoff-Hilfsstoff-Mischung zu dem Schmelzpunkt des Wirkstoffs als Punkt X aufgetragen ist, die Distanz c zwischen diesem Punkt X und dem Punkt 1/1 ≥ 0,08 ist.

11. Pharmazeutische Formulierung nach Anspruch 10, wobei der Hilfsstoff Eudragit E ist und der Wirkstoff ausgewählt ist aus der Gruppe bestehend aus Naproxen, lbuprofen, Oxcarbazepin, Paracetamol, Lidocain und Etofyllin.

12. Pharmazeutische Formulierung nach Anspruch 10, wobei der Hilfsstoff Copovidon ist und der Wirkstoff ausgewählt ist aus der Gruppe bestehend aus Paracetamol, Naproxen, Celecoxib und Etofyllin.

13. Pharmazeutische Formulierung nach Anspruch 10, wobei der Hilfsstoff Isomalt ist und der Wirkstoff ausgewählt ist aus der Gruppe bestehend aus Paracetamol und Etofyllin.
